# EUROPEAN PATENT SPECIFICATION

(11) **EP 0 671 933 B1**
(45) Date of publication and mention of the grant of the patent: **04.02.1998**
(21) Application number: 93920050.7
(22) Date of filing: 18.08.1993
(51) Int. Cl.: A61K 38/20

(54) **NOVEL USES OF IL-10**
NEUE VERWENDUNGEN FÜR IL-10
NOUVELLES UTILISATIONS D'IL-10

(30) Priority: 20.08.1992 US 932900; 21.08.1992 US 933419; 21.08.1992 US 933462; 21.08.1992 US 933950
(43) Date of publication of application: 20.09.1995
(60) Divisional application: 97108651.7
(73) Proprietor: SCHERING CORPORATION, Kenilworth New Jersey 07033 (US)
(72) Inventor: COFFMAN, Robert, Portola Valley, CA 94025 (US); DE VRIES, Jan, E., Los Altos, CA 94022 (US); DE WAAL MALEFYT, Rene, Sunnyvale, CA 94086 (US); POWRIE, Fiona, Mountain View, CA 94040 (US); RENNICK, Donna, Los Altos, CA 94022 (US)
(74) Representative: Ritter, Stephen David
(86) International application number: US9307646
(87) International publication number: WO9404180

(56) References cited:
- EP-A- 0 405 980
- WO-A-92/12725
- JOURNAL OF CELLULAR BIOCHEMISTRY, SUPPL. 0, (16 PART C) 21 February 1992 , NEW YORK, N.Y., US page 74 C. FLUCKIGER ET AL. 'BIOLOGIC EFFECTS OF IL-10 ON CHRONIC LYMPHOCYTIC LEUKEMIA CELLS'
- IMMUNOLOGY vol. 72, no. 2 , February 1991 , OXFORD, GB pages 161 - 166 C. BELLO-FERNANDEZ ET AL. 'IL-4 ACTS AS A HOMEOSTATIC REGULATOR OF IL-2-INDUCED TNF AND IFN-GAMMA.'
- JOURNAL OF IMMUNOLOGY. vol. 145, no. 9 , 1 November 1990 , BALTIMORE US pages 2938 - 2945 T.R. MOSMANN ET AL. 'ISOLATION OF MONOCLONAL ANTIBODIES SPECIFIC FOR IL-4, IL-5, IL-6, AND A NEW Th2-SPECIFIC CYTOKINE (IL-10), CYTOKINE SYNTHESIS INHIBITORY FACTOR, BY USING A SOLID PHASE RADIOIMMUNOADSORBENT ASSAY.'
- INTERNATIONAL ARCHIVES OF ALLERGY AND IMMUNOLOGY vol. 99, no. 1 , 17 July 1992 , BASEL, CH pages 8 - 15 H. SPITS ET AL. 'FUNCTIONAL CHARACTERIZATION OF HUMAN IL-10.'
- JOURNAL OF CLINICAL IMMUNOLOGY vol. 12, no. 4 , July 1992 , NEW YORK, N.Y., US pages 239 - 247 M. HOWARD ET AL. 'BIOLOGICAL PROPERTIES OF INTERLEUKIN 10.'
- CHEMICAL ABSTRACTS, vol. 119, no. 23, 6 December 1993, Columbus, Ohio, US; abstract no. 247336s, D. RENNICK ET AL 'INTERLEUKIN 10 AND THE INFLAMMATORY RESPONSE.' page 737 ;
- GASTROENTEROLOGY vol. 104, no. 4 SP , April 1993 , NEW YORK, N.Y., US page A751 G.E. MULLIN ET AL. 'ABNORMAL IL-10 mRNA PRODUCTION IN THE INTESTINAL MUCOSAL LESIONS OF INFLAMMATORY BOWEL DISEASE.'

## Description

The present invention relates to compositions for treating inflammatory bowel disease.

The immune system carries out a number of natural and adaptive immune reactions, two broad categories of which are called humoral and cellular immune responses. Humoral immunity refers broadly to antibody production and actions by B-cells including plasma cells. Cellular immunity is mediated by cells including T-cells, monocytes, macrophages and histiocytes.

T-cells may be further categorized according to various functions or markers. For instance, T-cells can be classified as T helper cells or T suppressor cells. Additionally, T-cells can be activated to become cytotoxic or to perform other more specialized functions. Normally, T-cells and B-cells have interactions that may regulate each other's activity to some extent. See, e.g., Paul (ed.) *Fundamentals of Immunology* (2d ed.) Raven Press, New York (1989).

For different antigens either cellular or humoral responses may predominate, typically in a mutually exclusive fashion. The severity of some diseases, e.g., leprosy, leishmaniasis and some types of autoimmunity may be due inappropriate dominance of one class of response over the other. Mosmann *et al., Immunol. Today 8*:223 (1987); Mosmann *et al., Ann. Rev. Immunol. 7*:145 (1989); Parish, *Transplant. Rev 13*:35 (1972); and Liew, *Immunol. Today 10*:40 (1989).

One of the mechanisms by which the immune system is regulated involves the production of proteins called cytokines. Lymphokines are cytokines produced by T-cells and some B-cells, and monokines are cytokines produced by monocytes. Cytokines, which may be glycosylated, mediate numerous immune responses.

IL-4 is a cytokine capable of stimulating production of antibody producing B-cells, which also promotes growth of killer T-cells or cytotoxic T-cells. IL-4 can also inhibit the activity of T-helper cells type 1 (Th1). This in turn may inhibit production of more B-cells or antibody production by more B-cells. Thus, IL-4 is part of an internal regulatory mechanism.

IL-10 is a cytokine capable of mediating a number of actions or effects. IL-10 has been isolated from both mouse and human cells and is involved in controlling the immune responses of different classes or subsets of T helper (Th) cells. Th cells can be divided into different subsets that are distinguished by their cytokine production profiles.

Th1 T cell clones produce interleukin-2 (IL-2) and IFN-γ, whereas Th2 cell clones secrete IL-10, IL-4 and interleukin-5 (IL-5), generally following activation by antigens or mitogenic lectins. Both classes of Th cell clones produce cytokines such as tumor necrosis factor-α (TNF-α), interleukin-3 (IL-3), and granulocyte-macrophage colony stimulating factor (GM-CSF). A third category of Th cells (Th0) produces IL-2, IFN-γ, IL-4, IL-5, TNF-α, IL-3 and GM-CSF simultaneously.

The different cytokine production patterns of Th1 and Th2 cells reflect their helper functions. Th1 cells are predominantly involved in delayed-type hypersensitivity responses, whereas Th2 cells are associated with antibody production. Since antibody (Th2 pathways) and delayed-type hypersensitivity (Th1 pathways) responses are often mutually exclusive, Th1 and Th2 cells are thought to have cross-regulatory effects. IFN-γ produced by Th1 cells inhibits proliferation of Th2 cells, and IL-10 produced by Th2 cells inhibits cytokine synthesis by Th1 cell clones, especially IFN-γ and IL-2 production.

Inflammatory bowel disease (IBD) refers to a group of gastrointestinal disorders characterized by a chronic non-specific inflammation of portions of the gastrointestinal tract. Ulcerative colitis and Crohn's Disease, the most prominent examples of IBD in humans, are associated with many symptoms and complications, including growth retardation in children, rectal prolapse, blood in stools (e.g., melena and/or hematochezia), wasting, iron deficiency, and anemia, e.g., iron deficiency anemia and anemia of chronic disease or of chronic inflammation.

The etiology of IBD is unclear. See, Wyngaarden and Smith (eds.) *Cecil's Textbook of Medicine* (W.B. Saunders Co. 1985), Berkow (ed.) *The Merck Manual of Diagnosis and Therapy* (Merck Sharp & Dohme Research Laboratories, 1982), and *Harrison's Principles of Internal Medicine,* 12th Ed., McGraw-Hill, Inc. (1991).

Ulcerative colitis is a chronic, non-specific, inflammatory, and ulcerative disease having manifestations primarily in the colonic mucosa. It is frequently characterized by bloody diarrhea, abdominal cramps, blood and mucus in the stools, malaise, fever, anemia, anorexia, weight loss, leukocytosis, hypoalbuminemia, and an elevated erythrocyte sedimentation rate (ESR). Complications can include hemorrhage, toxic colitis, toxic megacolon, occasional rectovaginal fistulas, and an increased risk for the development of colon cancer.

Ulcerative colitis is also associated with complications distant from the colon, such as arthritis, ankylosing spondylitis, sacroileitis, posterior uveitis, erythema nodosum, pyoderma gangrenosum, and episcleritis. Treatment varies considerably with the severity and duration of the disease. For instance, fluid therapy to prevent dehydration and electrolyte imbalance is frequently indicated in a severe attack. Additionally, special dietary measures are sometimes useful. Medications include various corticosteroids, sulphasalazine and some of its derivatives, and possibly immunosuppressive drugs.

Crohn's Disease shares many features in common with ulcerative colitis. Crohn's Disease is distinguishable in that lesions tend to be sharply demarcated from adjacent normal bowel, in contrast to the lesions of ulcerative colitis which are fairly diffuse. Additionally, Crohn's Disease predominately afflicts the ileum (ileitis) and the ileum and colon (ileocolitis). In some cases, the colon alone is diseased (granulomatous colitis) and sometimes the entire small bowel is involved (jejunoileitis). In rare cases, the stomach, duodenum, or esophagus are involved. Lesions include a sarcoid-type epithelioid granuloma in roughly half of the clinical cases.

Lesions of Crohn's Disease can be transmural, including deep ulceration, edema and fibrosis which can lead to obstruction and fistula formation as well as abscess formation. This contrasts with ulcerative colitis which usually yields much shallower lesions, although occasionally the complications of fibrosis, obstruction, fistula formation, and abscesses are seen in ulcerative colitis as well. Current treatment is similar for both diseases and includes the use of steroids, sulphasalazine and its derivatives, and immunosuppressive drugs such as cyclosporin A, mercaptopurine and azathioprine, all of which can produce strong undesired side effects.

Because of the medical importance of inflammatory bowel disease, there is a need for improved compositions and methods for treating this condition.

### SUMMARY OF THE INVENTION

The present invention fills the foregoing needs by providing compositions and methods for treating the above-mentioned condition.

More particularly, this invention provides the use of IL-10 in the manufacture of a pharmaceutical composition for treating inflammatory bowel disease.

Preferably, human IL-10 is used in the foregoing method for the treatment of human beings.

### DESCRIPTION OF THE INVENTION

All nucleic acid sequences disclosed follow the normal 5' to 3' convention, as read from left to right. Standard single-letter abbreviations are used for the nucleotide bases in the sequences (37 C.F.R. § 1.822).

### Sources of Cytokines and Antibodies

As used herein, "interleukin-10" or "IL-10" is defined as a protein which (a) has an amino acid sequence of mature (e.g., lacking a secretory leader sequence) IL-10 substantially as disclosed in International Application No. WO 91/00349, and (b) has biological activity that is common to native IL-10. Both glycosylated (e.g. produced in eukaryotic cells such as CHO cells) and unglycosylated (e.g., chemically synthesized by standard methods or produced in *E. coli*) IL-10 can be used. Also included are muteins and other analogs, including BCRF1 (Epstein Barr Virus viral IL-10; also called simply viral IL-10) protein, which possess the biological activity of IL-10. International Application Publication No. WO 91/00349 describes a number of *in vitro* assays suitable for measuring IL-10 activity. A number of preferred modifications of IL-10 are described in this application.

IL-10 suitable for use in the invention can be obtained from a number of sources. For example, it can be isolated from culture media of activated T-cells capable of secreting the protein. Additionally, the IL-10 or active fragments thereof can be chemically synthesized using standard techniques as known in the art. See, e.g., Merrifield, Science *233*:341-47 (1986) and Atherton *et al., Solid Phase Peptide Synthesis, A Practical Approach,* 1989, I.R.L. Press, Oxford.

Recombinant human IL-10 is also an article of commerce, available for purchase, e.g., from PeproTech, Inc., Rocky Hill, NJ.

Methods for making recombinant human IL-10 are disclosed, e.g., in International Application Publication No. WO 91/00349. Clones comprising sequences encoding human IL-10 were deposited with the American Type Culture Collection (ATCC), Rockville, Maryland, on December 20, 1989 and assigned Accession Numbers 68191 and 68192. The cloning and expression of a viral IL-10 (BCRFI protein) from Epstein Bar virus has been disclosed by Moore *et al.* [*Science 248*:1230 (1990)].

Alternatively, recombinant IL-10 can be produced as described in International Application Publication No. WO 94/04180 for IL-4 using known human IL-10 or viral IL-10 sequence information.

Preferably, human IL-10 is used for the treatment of humans, although viral IL-10 or IL-10 from some other mammalian species can be used instead. Most preferably, the IL-10 used is recombinant human IL-10.

IL-10 from whatever source can be purified using standard methods including but not limited to acid or salt precipitation, ion-exchange chromatography, metal chelate chromatography, gel filtration, fast protein liquid chromatography (FPLC), high performance liquid chromatography (HPLC), preparative disc gel or curtain electrophoresis, isoelectric focusing, low temperature organic solvent fractionation, countercurrent distribution and immunoaffinity chromatography.

Pharmaceutical compositions of the invention are suitable for use in a variety of drug delivery systems. For a brief review of present methods for drug delivery, see Langer, *Science 249*:1527 (1990). Methods for preparing administrable compounds will be known or apparent to those skilled in the art and are described in more detail in for example, *Remington's Pharmaceutical Science,* 17th ed., Mack Publishing Company, Easton, PA (1985).

The IL-10 used in this invention may be prepared as formulations in pharmaceutically acceptable media, for example, saline, phosphate buffered saline (PBS), Ringer's solution, dextrose/saline, Hank's solution, and glucose. The compositions may contain pharmaceutically acceptable auxiliary substances as required to approximate physiological conditions, such as buffering agents, tonicity adjusting agents, wetting agents, detergents, and the like. Additives may also include additional active ingredients, e.g., bactericidal agents, or stabilizers. The amount administered to the patient will vary depending upon what is being administered, the purpose of the administration, such as prophylaxis or therapy, the state of the host, the manner of administration, and the like.

The pharmaceutical compositions are typically intended for transdermal or parenteral administration, *e.g.,* intravenously, subcutaneously, or intramuscularly. Orally administrative forms are also desired and can be provided by modifying the composition to bypass the stomach environment. The composition can be used for prophylactic and/or therapeutic treatment. Preferably, the pharmaceutical compositions are administered intravenously. Thus, the invention provides compositions which comprise an IL-10 polypeptide dissolved or suspended in an acceptable carrier, preferably an aqueous carrier. These compositions may be sterilized by conventional sterilization techniques, or may be sterile filtered.

The resulting aqueous solutions may be packaged for use as is, or lyophilized, the lyophilized preparation being combined with a sterile aqueous carrier prior to administration. The IL-10 may also be administered with a second biologically active agent, such as a standard chemotherapeutic agent. Such agents include but are not limited to vincristine, daunorubicin, L-asparaginase, mitoxantrone and amsacrine.

In therapeutic applications, the pharmaceutical compositions are administered to a patient in an amount sufficient to to produce the desired effect, defined as a "therapeutically effective dose." The therapeutically effective dose of IL-10 will vary according to, for example, the particular use for which the treatment is made, the manner of administration, the health and condition of the patient, and the judgment of the prescribing physician. For example, the dose for continuous infusion will typically be the range of about 500 ng to about 800 µg per day for a 70 kg patient, preferably between about 10 µg and about 300 µg. The dose will typically be between 700 ng/kg/day and 16 µg/kg/day.

The concentration of IL-10 in the pharmaceutical formulations can vary widely, *i.e.,* from about 10 µg to about 5 mg/ml, preferably between about 100 µg and about 2 mg/ml. The concentration will usually be selected primarily by fluid volumes, viscosities, etc., in accordance with the particular mode of administration selected. Thus, a typical pharmaceutical composition for intravenous infusion could be made up to contain 250 ml of dextrose/saline solution and 2.5 mg IL-10.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating normally employed excipients, such as those carriers previously listed, and generally 10-95% of active ingredient, that is, an IL-10 polypeptide of the invention, preferably 25%-75%.

For aerosol administration, the IL-10 is preferably supplied in finely divided form along with a surfactant and propellant. Typical percentages of IL-10 are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride such as, for example, ethylene glycol, glycerol, erythritol, arbitol, mannitol, sorbitol, the hexitol anhydrides derived from sorbitol, and the polyoxyethylene and polyoxypropylene derivatives of these esters. Mixed esters, such as mixed or natural glycerides may be employed.

The surfactant may constitute 0.1%-20% by weight of the composition, preferably 0.25-5%. The balance of the composition is ordinarily propellant. Liquified propellants are typically gases at ambient conditions, and are condensed under pressure. Among suitable liquified propellants are the lower alkanes containing up to 5 carbons, such as butane and propane; and preferably fluorinated or fluorochlorinated alkanes. Mixtures of the above may also be employed. In producing the aerosol, a container equipped with a suitable valve is filled with the appropriate propellant, containing the finely divided peptide(s) and surfactant. The ingredients are thus maintained at an elevated pressure until released by action of the valve.

To enhance the serum half-life, the IL-10 may be encapsulated, introduced into the lumen of liposomes, prepared as a colloid, or other conventional techniques may be employed which provide an extended lifetime of the peptides. Thus, in certain embodiments, the IL-10 may be encapsulated in a liposome. A variety of methods are available for preparing liposomes, as described in, e.g., Szoka *et al., Ann. Rev. Biophys. Bioeng. 9*:467 (1980), U.S. Pat. Nos. 4, 235,871, 4,501,728, and 4,837,028.

Following preparation, the liposomes may be sized to achieve a desired size range and relatively narrow distribution of liposome sizes. Several techniques are available for sizing liposomes to a desired size, one of which is described in U.S. Patent No. 4,737,323.

Even under the most efficient encapsulation methods, the initial sized liposome suspension may contain up to 50% or more drug in a free (non-encapsulated) form. Several methods are available for removing non-entrapped compound from a liposome suspension. In one method, the liposomes in the suspension are pelleted by high-speed centrifugation leaving free compound and very small liposomes in the supernatant. Another method involves concentrating the suspension by ultrafiltration, then resuspending the concentrated liposomes in a replacement medium. Alternatively, gel filtration can be used to separate large liposome particles from solute molecules.

Following the above treatment, the liposome suspension is brought to a desired concentration for use in intravenous administration. This may involve resuspending the liposomes in a suitable volume of injection medium, where the liposomes have been concentrated, for example by centrifugation or ultrafiltration, or concentrating the suspension, where the drug removal step has increased total suspension volume. The suspension is then sterilized by filtration as described above. The liposomes comprising the peptides of the invention may be administered parenterally as described above.

### Inflammatory Bowel Disease

The term "inflammatory bowel disease," or "IBD," is used herein to include ulcerative colitis and Crohn's Disease. The administration of IL-10 to treat IBD is preferably parenteral, such as intravascular. Most preferably, the administration is intravenous and the mammal treated is a human being.

The amount of IL-10 administered will generally range from about 1 µg to about 100 mg per kilogram of body weight. Often, the range will be from about 10 µg to about 1000 µg per kilogram of body weight. Most preferably, a dose of from about 50 µg to about 100 µg per kilogram of body weight will be administered.

The IL-10 can be administered alone or co-administered in combination with one or more additional therapeutic agents. Examples of such agents useful in controlling episodic inflammation of intestinal tissue include corticosteroids, sulphasalazine, derivatives of sulphasalazine, immunosuppresive drugs such as cyclosporin A, mercaptopurine, and azathioprine, and other cytokines. The co-administration can be sequential or simultaneous. Co-administration generally means that the multiple (two or more) therapeutics are present in the recipient during a specified time interval. Typically, if a second agent is administered within a half-life of a first agent, the two agents are considered co-administered.

This invention further provides a method for predicting a mammal's predisposition for development of an inflammatory condition characterized by suboptimal levels of IL-10 comprising assaying a sample taken from the mammal for an IL-10 level. Suboptimal levels include undetectable amounts. A detectable level could be compared to a known normal level of IL-10. Alternatively, one can assay for inflammatory mediators such as IL-1, IL-6, TNF, and IFN-γ using commercially available diagnostic kits.

Overproduction of one of these mediators can indicate that insufficient amounts of IL-10 are available. Preferably, blood is the sample source. The method allows for prediction of predisposition to a number of inflammatory conditions, such as an IBD or an anemia, an arthritis, a dermatitis, or other syndrome associated with an IBD.

Pharmaceutical compositions are also provided. A composition for administration to a mammal having an IBD, such as ulcerative colitis or Crohn's Disease, includes an amount of IL-10 effective to ameliorate at least one symptom or sign of the IBD in the mammal, and a pharmaceutically acceptable carrier as described above.

Generally, the term "symptom refers any subjective evidence of disease or of a patient's condition. This includes evidence as perceived by the patient. Examples of symptoms of IBD include diarrhea, abdominal pain, fever, melena, hematochezia, and weight loss. The term "sign" refers generally to any objective evidence of a disease or condition, usually as percieved by an examining physician or features which would reveal themselves on a laboratory evaluation or other tests such as an ultrasonic study or a radiographic test.

Some examples of signs of IBD include abdominal mass, glossitis, aphthous ulcer, anal fissure, perianal fistula, anemia, malabsorption, and iron deficiency. Occasionally, signs and symptoms overlap. For example, the patient complains of bloody stools (a symptom), and a laboratory test of a stool sample is positive for blood (a sign).

Pharmaceutical compositions of this embodiment of the invention are preferably in a form suitable for parenteral administration. Preferably, the effective amount is a unit dose presented in an ampoule. Alternatively, the effective amount could be presented in a vial containing multiple doses or it could be offered in some other form. Examples of pharmaceutically acceptable additives include vehicles such as aqueous vehicles, buffers, diluents, antimicrobials, and preservatives.

Biological assays of the cytokines themselves can also be used to determine IL-10 activity. A biological assay for human lymphotoxin is disclosed in Aggarwal, *Methods in Enzymology 116*:441 (1985) and Matthews, *et al.,* (Clemens, *et al.,* eds.), *Cytokines and Interferons; A Practical Approach*: pp. 221-225 (IRL Press, Washington, D.C. 1987). Human IL-2 and GM-CSF can be assayed with factor-dependent cell lines CTLL-2 and KG-1, available from the ATCC under accession numbers TIB 214 and CCL 246, respectively. Human IL-3 can be assayed by its ability to stimulate the formation of a wide range of hematopoietic cell colonies in soft agar cultures, e.g., as described by Metclaf, "The Hemopoietic Colony Stimulating Factors" (Elsevier, Amsterdam, 1984). IFN-γ can be quantified with anti-viral assays, e.g., Meager in Clemens, *et al.* (eds.) at pp. 129-147. Monitoring of these cytokines can provide useful information on when an effective amount of IL-10 has been administered. Immunochemical assays such as ELISA can also be used.

The IL-10 may be administered in aqueous vehicles such as water, saline or buffered vehicles with or without various additives and/or diluting agents. Alternatively, a suspension, such as a zinc suspension, can be prepared to include the IL-10. Such a suspension can be useful for subcutaneous (SQ) or intramuscular (IM) injection. The proportion of IL-10 and additive can be varied over a broad range so long as both are present in effective amounts. On a per-dose basis, the amount of the IL-10 could range from about 1 microgram (µg) to about 100 milligrams (mg).

The total daily dose typically ranges from about 1 µg to about 100 mg per kilogram of body weight. Preferably, the dose is selected from a range of about 10 µg to about 1000 µg per kilogram of body weight. More preferably, the dose is selected from a range of about 50 µg to about 100 µg per kilogram of body weight. Dosages are administered on a schedule which produces the desired therapeutic effect and can be periodic over a short or longer term, or irregular as dictated by the episodic nature of such inflammations.

Compositions may be ingested orally or injected into the body. Formulations for oral use include compounds to protect the polypeptides from proteases which occur in the gastrointestinal tract. Injections are usually intramuscular, subcutaneous, intradermal or intravenous. Alternatively, intra-articular injection or other routes could be used in appropriate circumstances. Additionally, compositions including the IL-10 may be implanted into a patient or injected using a drug delivery system. See, for example, Urquhart, *et al., Ann. Rev. Pharmacol. Toxicol.* 24:199 (1984); Lewis, ed. "Controlled Release of Pesticides and Pharmaceuticals" (Plenum Press, New York, 1981); U.S., Patents Nos. 3,773,919 and 3,270,960.

Preferably, the peptide is administered parenterally, and preferably in a unit dosage injectable form. Examples of an injectable form include solutions, suspensions and emulsions. More preferably, an effective amount of IL-10 is administered intravenously.

The phrase "effective amount" is defined herein to mean an amount sufficient to ameliorate a symptom or sign of an autoimmune condition or of an undesirable or inappropriate inflammatory or immune response. Typical mammalian hosts will include mice, rats, cats, dogs, and primates, including human beings. An effective amount for a particular patient may vary depending on factors such as the condition being treated; the overall health of the patient; the method, route and dose of administration, and the severity of side affects.

Determination of the appropriate dose is made by the clinician using parameters known in the art. Generally, the dose begins with an amount somewhat less than the optimum dose and it is increased by small increments thereafter until the desired or optimum effect is achieved. *See* generally *The Merck Manual* § 269 "Pharmacokinetics and Drug Administration." TNFα, IFN-γ, IL-1, and IL-6 levels would be important indicators of when an effective dose is reached. Preferably, IL-10 derived from the mammalian species treated will be used.

Typically, the IL-10 is injected in association with a pharmaceutical carrier such as normal saline, Ringer's solution, dextrose solution, and other aqueous carriers known in the art. Appropriate non-aqueous carriers may also be used and examples include fixed oils and ethyl oleate. A preferred carrier is 5% dextrose in saline. Frequently, it is desirable to include additives in the carrier such as buffers and preservatives or other substances to enhance isotonicity and chemical stability.

The total daily dose may be given as a single injection or a continuous infusion. Alternatively, it may be divided into several smaller doses for bolus intravenous administration or administration by some other route such as intramuscular injection. Preferably, the IL-10 is administered as an intravenous bolus.

The IL-10 may be administered alone or in combination with other therapeutic agents. Examples of such agents in an inflammatory bowel indication include corticosteroids, sulphasalazine, derivatives of sulphasalazine, and selected cytotoxic drugs such as cyclosporin A, mercaptopurine, and azathioprine. Typically, the multiple medications are separately infused or injected sequentially. In appropriate circumstances, multiple medications are mixed and infused or injected simultaneously together, e.g., IL-10 with other cytokines, steroids, or other therapeutic reagents.

It should be noted that IBD symptoms may be episodic, so effective treatment may be achieved by administration of small doses at opportune moments. Alternatively, small amounts of continuous administration may provide long periods of episode-free health for suffering target animals.

The co-administration can be simultaneous or sequential. Generally, "co-administering" means that the cytokines are both present in the recipient during a specified time interval. Typically, if a second cytokine is administered within a half life of the first cytokine to be administered, the two cytokines were co-administered. Preferably, the co-administration is parenteral, and more preferably it is intravenous. The effective amount is selected from a range from about 15 µg to about 1500 µg per kilogram of body weight of the mammal.

### Inflammatory Bowel Disease

A murine model of IBD has been developed. This model contributes to elucidation of the etiology or mechanism of IBD. Additionally, it provides an experimental forum for various therapeutic agents. To develop the model, the IL-10 gene is either removed or rendered ineffective or non-expressible in the experimental animal. The animal is referred to as having the IL-10 gene "knocked out" or as being a "knockout" (KO) animal. The KO can be accomplished by any of several means known in the art.

For example, a transgenic animal whose IL-10 gene has been inactivated can be produced. *See* Goodnow, C., "Transgenic Animals," 1502-1504, *Encyclopedia of Immunology,* Roitt (ed.), Academic Press, San Diego (1992). Briefly, purified DNA is microinjected into the pronuclei of fertilized oocytes. Afterwards, the oocytes are surgically implanted into the oviducts of foster mothers. Alternatively, standard genetic breeding techniques may be used to isolate a homozygous mutant animal strain.

Additionally, there are methods which inactivate specific genes in the germ line of mice. In this case, a mutant transgene is introduced in place of the wild-type gene through homologous recombination. Embryonal stem (ES) cell lines "can be isolated and culture *in vitro* from mouse blastocysts, and later reimplanted into another blastocyst so as to partly colonize both the somatic and germ-line tissues of the resulting mouse." Goodnow at 1503.

DNA is introduced in the ES cells in culture and the blastocysts are surgically implanted in a foster mother. Heterozygous mutant progeny result which are mated to each other so that, ultimately, offspring having two mutant alleles, e.g., a homozygous mutant, are produced. These offspring would have no functional alleles of the gene of interest and allow for "genetic dissection" to remove specific genes. Analysis of the resulting offspring can provide insight into the role of the targeted gene in the KO mice in causing resultant phenotypes.

For additional methods of creating an experimental animal having deletion of a particular gene, *see* McMahon, *et al.,* "The Midbrain-Hindbrain Phenotype of *Wnt-1*^{*-*}*/Wnt-1*^{*-*} Mice Results from Stepwise Depletion of *engrailed*-Expressing Cells by 9.5 Days Postcoitum," *Cell 69*:581 (1992) and Travis, "Scoring a Technical Knockout in Mice," *Science 256:*1392 (1992). Additional applicable technologies include the art of mutagenesis and antisense technologies. *See* Goodnow, C. (1992, *supra*).

### Example 1 -- Murine Model for Inflammatory Bowel Disease.

Mice which were devoid of the IL-10 gene were raised. See Kuhn *et al. Science 254*:707 (1991); Capecchi, *Science* *244*:1288 (1989); Robertson (ed.) *Teratocarcinomas and embryonic stem cells: A Practical Approach,* I.R.L. Press, Oxford (1987); and Zijestra, *et al. Nature 336*:348 (1989), for descriptions of the general technology and for the procedures to make mice devoid of a selected gene. Mice which lack the IL-10 gene are referred to below as IL-10T mice, or knock out (KO) mice.

First Group. Four mice were sacrificed for bone marrow assays. A portion of their marrow was analyzed to determine their ability to produce myeloid and erythroid progenitors (see Table 1) and to assess production of stem cells (see Table 2). A remaining portion of the marrow was used for long-term bone marrow cultures to assess the capability of micro-environmental cells to support normal hematopoietic generation. The thymus and spleen contained normal numbers of T-cells and normal numbers of B-cells. The short-term progenitor and stem-cell assays are given in tabular form below. The cells from the controls and the knock-out mice were separately pooled.

**Table 1**

| Number of Colony-Forming Cells/Femur | | | | | | |
|---|---|---|---|---|---|---|
| | Total cells/femur | GM | BFU-e | CFU-e | Meg | Meg/Mix |
| Control | 9.5x10⁶ | 18,430 | 475 | 42,500 | 2,470 | 285 |
| IL-10T | 9.4x10⁶ | 21,902 | 940 | 59,740 | 2,271 | 658 |
| GM = granulocytes and macrophages; BFU = blood forming units; CFU = colony forming units; -e = erythroid; Meg = megakaryocyte colonies; Meg/Mix = megakaryocytes mixed with other lineages.Colony-stimulating factors used in assay: IL-3 + IL-6 + erythropoietin (Epo) | | | | | | |

**Table 2**

| Numbers of Day-14 CFU-S | |
|---|---|
| Control group | 1,678 per femur |
| IL-10T group | 1,517 per femur |
| Stem cell production is the number of CFU-S in a femur of the mouse. CFUS measures the formation of hemopoietic nodules in the spleen of lethally irradiated mice following transplantation. See Till *et al., Radiation Research 14*:213 (1961). | |

The IL-10T mice did not show any obvious defect in the production of myeloid, erythroid and stem cells. Concurrently, the long-term cultures had formed a layer of supportive stroma in all cultures including those from IL-10T mice. These cultures were set up in two stages: First, cells were put into culture to form the complex stromal layers needed to support continuous hematopoiesis. Then the cultures were reseeded with additional bone marrow cells from equivalent types of donors to provide the actual precursors that associate with the stromal cells. This second stage was undertaken when the second and third groups of mice were available.

Second and third groups. The bone marrow cells from these animals were needed to complete the long-term bone marrow culture experiments. In addition to using their bone marrow for this purpose, other procedures were performed (see Tables 3-7). The IL-10T animals were anemic but seemed to have normal levels of bone marrow precursors. Therefore, their anemia was evaluated and their organs were observed histologically.

**Table 3**

| Peripheral Blood Counts | | | | |
|---|---|---|---|---|
| Mice | | WBC/ml x 10⁶ | RBC/ml x 10⁹ | Platelets/ml x 10⁸ |
| Control | # 1 | 7.2 | 7.3 | 12.6 |
| | # 2 | 11.2 | 5.6 | 10.7 |
| | # 3 | 10.0 | 6.7 | 8.5 |
| | # 4 | 8.2 | 5.6 | 9.8 |
| | # 5 | 7.7 | 5.6 | 8.0 |
| IL-10T | # 1 | 4.0 | 2.4 | 20.6 |
| | # 2 | 4.8 | 7.1 | 8.0 |
| | # 3 | 7.5 | 3.9 | 27.3 |
| | # 4 | 6.5 | 5.5 | 23.9 |
| WBC = white blood cells, RBC = red blood cells | | | | |

**Table 4**

| Peripheral Blood Differentials | | | | | | |
|---|---|---|---|---|---|---|
| Mice | | % Lymphs | % Neut | % Mono | % Eos | % Retics |
| Control | # 1 | 87 | 12 | 1 | 0 | 2.4 |
| | # 2 | 72 | 26 | 2 | 0 | 1.2 |
| | # 3 | 80 | 18 | 1 | 1 | 0.6 |
| IL-10T | # 1 | 36 | 64 | 0 | 1 | 3.8 |
| | # 2 | 40 | 57 | 0 | 3 | 5.3 |
| | # 3 | 23 | 77 | 0 | 0 | 17.1 |
| Lymphs = lymphocytes, Neut = neutrophils, Mono = monocytes, Eos = eosinophils, Retics = reticulocytes | | | | | | |

RBC Morphology. All control animals had normal appearing red blood cells (RBC's). The IL-10T mice showed variable morphologies. IL-10T #1 had microcytic cells; #2 and #3 had microcytic cells plus some macrocytic, polychromic cells and occasional nucleated red blood cells (NRBC's). Red blood smears were stained with new methylene blue and the cells showing ribosomal staining were enumerated. Positive staining occurs in very young red blood cells (reticulocytes) and implies premature release from hemopoietic organs. This is referred to as the reticulocyte count.
Organ Histology of IL-10T Mice. Inspection of sectioned heart, lung, kidney and thymus appeared normal. The liver appeared normal except for occasional foci of mononuclear and neutrophilic granulocytes. The spleens were grossly abnormal. Follicles were present but the red pulp area was filled with hematopoietic cells, mostly NRBC's and blast forms (immature cells). There were few or no mature RBC's and there was no iron storage with the macrophages.

**Table 5**

| Spleen Cell Differentials | | | | | | | | | |
|---|---|---|---|---|---|---|---|---|---|
| Mice | | Cells/spleen | (% of total cells) | | | | | | |
| | | | Lymph | Mono | Neu | Eos | Plasma Cell | Blast | Nuc RBC |
| Control | # 1 | 1.5x10⁸ | 88 | 2 | 1 | <1 | <1 | 1 | 6 |
| | # 2 | 1.5x10⁸ | 89 | 1 | <1 | <1 | <1 | 2 | 7 |
| | # 3 | 1.1x10⁸ | 79 | 3 | 4 | 1 | 2 | 2 | 8 |
| IL-10T | # 1 | 2.1x10⁸ | 64 | 2 | 7 | 1 | 1 | 2 | 22 |
| | # 2 | 2.5x10⁸ | 54 | 2 | 13 | 3 | 1 | 6 | 21 |
| | # 3 | 1.6x10⁸ | 43 | 2 | 10 | 1 | 1 | 10 | 32 |
| Lymph = lymphocytes, Mono = monocytes, Neu = neutrophils, Eos = eosinophils, Retics = reticulocytes, Blast = blastocyte, Nuc RBC = nucleated red blood cell | | | | | | | | | |

**Table 6**

| Bone Marrow Differentials | | | | | | | | | |
|---|---|---|---|---|---|---|---|---|---|
| Mice | | (% of Total) | | | | | | | |
| | | Neu | Neu MB | Eos | Eos MB | Nuc RBC | Plasma Cell | Lym | Blast Undif |
| Control | # 1 | 46 | 12 | 10 | 4 | 20 | <1 | 4 | 4 |
| | # 2 | 39 | 9 | 5 | 3 | 37 | <1 | 5 | 2 |
| | # 3 | 35 | 10 | 12 | 4 | 29 | <1 | 6 | 2 |
| IL-10T | # 1 | 46 | 12 | 7 | 2 | 28 | <1 | 2 | 2 |
| | # 2 | 56 | 19 | 4 | 1 | 18 | <1 | 1 | 1 |
| | # 3 | 53 | 14 | 2 | 1 | 27 | 1 | 1 | 1 |
| Neu MB = neutrophilic myeloblast; Eos MB = eosinophilic myeloblast; Nuc RBC = nucleated RBC; Blast Undif = primitive undifferentiated blastocyte; Lym = lymphocytes | | | | | | | | | |

The following data are the results of colony-forming assays. The assays were done with spleen cells as well as bone marrow cells. IL-3 + c-kit ligand instead of IL-3 + IL-6 was used to stimulate the colony-forming cells. The combination of IL-3 + c-kit ligand is more effective in stimulating erythroid precursors as evidenced by comparing the numbers of BFU-e obtained in the first assay (above) with the numbers of BFU-e obtained in the following assays.

**Table 7**

| Colony-Forming Assays | | | | | | | |
|---|---|---|---|---|---|---|---|
| Mice | GM | GEM | GEMM | BFU-e | CFU-e | MEG | MEG/MIX |
| | | | | | | | |

| Spleen (colonies/spleen) | | | | | | | |
|---|---|---|---|---|---|---|---|
| C1 | 18,535 | 618 | 309 | 9,268 | 42,322 | 618 | 1,236 |
| C2 | 7,736 | 893 | 595 | 2,678 | 44,926 | 1,488 | 893 |
| C3 | 8,670 | 650 | 650 | 7,152 | 71,741 | 433 | 433 |
| K1 | 55,020 | 8,400 | 3,360 | 51,660 | 393,120 | 2,100 | 6,300 |
| K2 | 111,060 | 25,746 | 10,601 | 96,925 | 498,762 | 14,135 | 12,116 |
| K3 | 68,274 | 20,096 | 9,890 | 103,050 | 1,012,633 | 14,357 | 9,252 |

| Bone Marrow (colonies/femur) | | | | | | | |
|---|---|---|---|---|---|---|---|
| C1 | 37,300 | 3,632 | 838 | 7,963 | 35,065 | 978 | 419 |
| C2 | 49,159 | 2,552 | 510 | 5,954 | 81,478 | 680 | 1,021 |
| C3 | 30,343 | 1,916 | 639 | 6,069 | 24,754 | 958 | 958 |
| K1 | 63,440 | 1,823 | 1,641 | 8,750 | 58,336 | 1,276 | 1,276 |
| K2 | 37,340 | 6,570 | 438 | 8,870 | 47,085 | 986 | 1,424 |
| K3 | 35,432 | 4,006 | 1,753 | 14,273 | 52,208 | 1,628 | 4,132 |
| C1, C2, and C3 refer to the same control mice also designated Control #1, #2, and #3. K1, K2, and K3 refer to the same mice also designated IL-10T #1, #2, and #3. GM = granulocyte, macrophage GEM = granulocyte, erythoid, macrophage GEMM = granulocyte, erythoid, macrophage, megakaryocyte | | | | | | | |

**Table 8**

| Occult Blood Tests | | | |
|---|---|---|---|
| Mice | | day 1 | day 2 |
| Control | # 1 | neg | neg |
| | # 2 | neg | neg |
| | # 3 | no sample | neg |
| IL-10T | # 1 | neg | neg |
| | # 2 | pos | pos |
| | # 3 | pos | pos |

Examination of Feces. Studies of the feces of the IL-10T mice did not reveal evidence of eggs suggesting parasitic infection. There was no evidence of mature parasites residing in the upper or lower intestine.

The animals varied between slightly and markedly anemic. The anemia cannot be explained by inability to generate erythrocytes. The IL-10T mice showed signs of trying to compensate for the anemia. First, they produced larger than normal numbers of RBC precursors in their spleens. In mice, the spleen normally would try to compensate for RBC deficiencies. The murine spleens were two times larger than in normal animals and they were overactive in trying to generate RBC's.

Some of the mice were compensating well. They had near normal numbers of RBC's in their blood and, they had not released many immature cells into circulation because their reticulocyte numbers were relatively normal. However, other mice fell behind and they pushed cells into the circulation prematurely which is why their reticulocyte counts were so high (i.e., 17%). They also had a few circulating nucleated RBC's.

These mice also had higher than normal platelet counts as well as elevated numbers of megakaryocytes in their spleens. This was a sign that they may have been bleeding. Loss of blood would account for the chronic anemia with the depletion of iron stores (this iron deficiency was evident in both the spleen and bone marrow compartments). Upon autopsy, it was clear that there was no internal bleeding. However, most of the IL-10T mice showed blood in their feces and also showed prolapsed rectums. The mice were examined closely and found to be negative for pinworms, tapeworms, or other parasitic infections. There was a direct correlation between positive blood in the feces and the severity of their anemia.

In addition to the anemia and bleeding problem, the IL-10T mice had more neutrophilic granulocytes in their blood than lymphocytes. This is the opposite of what is normal for mice. See the peripheral blood differential (Table 4). This observation is also consistent with a huge increase in myeloid precursors found in the spleen colony-forming assays Table 9). Thus, the mice suffered from the syndrome "anemia of chronic inflammation."

At autopsy, tissue sections prepared from IL- 10T mice and litter-mate controls were examined. All specimens from the controls appeared normal. Referring to the IL-10T mice, the thymus and spinal tissue appeared normal. The spleen showed mild to marked increase in erythroblasts and myeloblasts plus megakaryocytes. RBC pools were small or essentially nonexistent. Prussian blue staining showed depletion of iron stores and there was evidence of increased extramedullary hematopoiesis.

Depletion of iron stores is consistent with blood loss and results in abnormal erythropoiesis. On gross examination, the spleens were one and one-half to three times normal size. The mesenteric lymph nodes were very large compared to normal and were almost as large as a normal spleen. B and T-cell follicles were similarly enlarged, and this picture is consistent with an inflammatory response.

The small intestine showed well-formed mucosa and submucosa. In some sections, the numbers of polymorphonucleated white cells (PMN) and mononuclear cells were slightly increased. The large intestine and rectum revealed marked inflammation of the rectum extending proximally including the lower portion of the colon. A marked amount of edema and infiltrating cells were evident. PMN's were the predominant cell type, but eosinophils, lymphocytes, plasma cells, and epithelioid cells (cells which resemble epithelium) were present. Inflammation involved the mucosa, crypts and submucosa. Occasionally, infiltrates were found in the muscularis layer, especially in the internal sphincter region. In many areas, the epithelium was lost or absent. Multiple lesions showed fibrous material with aggregated RBC's and PMN's. Some blood vessels were congested with RBC's. Clumps of epithelioid cells suggested early formation of granuloma, but no well-formed granulomas were evident. There was no evidence of parasites, eggs or intracellular bacteria, which can cause chronic inflammation. The multiple lesions and hemorrhage were consistent with `the positive result of the stool occult blood test. The entire picture was consistent with IBD with secondary anemia and wasting.

### Example 2 -- IL-10 KO Mice Respond to Treatment With Exogenous IL-10.

Four mice which were devoid of the IL-10 gene (IL-10 KO) and three normal control mice were examined. The control mice had a normal physical appearance. All three of the control mice had negative occult blood stool samples and had normal-appearing rectums. Some of the IL-10 KO mice were noticeably smaller than the controls and they showed signs of poor health. They had ruffled hair and poor vigor compared to the normal controls. One of the four IL-10 KO mice was hunched and walked with an abnormal gait. All four of the IL-10 KO mice had swollen rectums and either erythematous rectal tissue or definite rectal prolapse.

Two of the experimental KO mice had definitely positive occult blood stool samples on two separate occasions, whereas the third mouse was negative on one occasion and equivocal on a second occasion. Rectal smears were performed on all four KO mice and three had purulent exudates with large numbers of neutrophilic granulocytes. Erythrocytes were also present on individual smears of the animal that was bleeding from the rectum at the time of sampling. Because IL-10 KO mice showed wasting with time, the weight of the IL-10 KO mice and the normal controls were recorded as part of the examination.

Three of the IL-10 mice were injected daily for two weeks with 35 micrograms of IL-10. These three mice had consistently shown purulent exudates (only 2 out of the 3 were also showing signs of bleeding and 1 out of the 3 was hunched up and walked abnormally). One IL-10 KO mouse (negative for bleeding and a purulent exudate) and the normal controls were injected daily for two weeks with tromethamine (TRIS) buffer. The general health, physical appearance, rectal exudate, occult blood and weight were monitored.

After the second week of IL-10 infusion, all three IL-10 KO mice showed increased vigor, smoothed hair, and normal posture and walk. All three showed reduced rectal swelling, although signs of inflammation were still present. All three had negative occult blood results and there were no signs of erythrocytes in their rectal smears. The amount of exudate was reduced; however, neutrophilic granulocytes were still observed in their rectal smears. The smear of one animal now showed more epithelial and lymphocyte cells than neutrophilic granulocytes. One animal maintained its weight at approximately 16 grams. The other two increased their weight from 16 to 17.8 grams and 11.4 to 14 grams.

The IL-10 KO mouse that received TRIS buffer for two weeks still had a negative occult blood stool sample. It showed abundant exudate which contained large numbers of neutrophilic granulocytes. The rectum was still swollen and susceptible to prolapse. The animal was hunched up and walked abnormally. It also showed obvious signs of wasting, and its weight had dropped from 16.6 to 14 grams. The normal controls which had also been injected with TRIS buffer retained a normal health appearance including their rectums. Their occult blood stool samples were still negative and there were no rectal exudates detected. Their weight increased from 17.1 to 19 grams and 17.4 to 18.7 grams. Even several weeks later, the animals continued to gain weight.

### Example 3 -- Amelioration of Signs or Symptoms of Attributable to Inflammatory Bowel Disease.

A human patient having IBD can be treated with IL-10 according to the invention as follows. Such a patient typically has symptoms which may include diarrhea, abdominal pain, fever, melena, hematochezia, and weight loss and signs which may include abdominal mass, glossitis, aphthous ulcer, anal fissure, perianal fistula, anemia, malabsorption, and iron deficiency. The patient is initially treated with five µg of IL-10 per kilogram body weight per day. Because the patient weighs 70 kg, the initial starting dose is 350 µg per day of IL-10. This dose is administered as an intravenous bolus. The dose is increased by about 50 to 150 µg per day depending on the patient's tolerance and response. An optimum dose of about 700 µg per day (10 µg per kilogram per day) is achieved after several days.

Prior to the first treatment, and daily thereafter until several days after the final treatment, the patient is monitored clinically and with laboratory parameters. The laboratory parameters include blood counts with particular attention to the total white blood cell count and its differential. Of special interest is whether the numbers of white cells remain normal or without significant change from the patient's pre-treatment level. With respect to the white cell differential, particular attention is given to whether immature forms are appearing in the peripheral blood and whether the normal ratio of different types of white cells is constant or changing. Special attention is paid to the ratio of lymphocytes and monocytes in the peripheral blood.

Additional parameters which can be monitored include the erythrocyte sedimentation rate (ESR), chemical profiles, blood levels of IL-6, TNF and IFN-γ. Finally, regular, such as daily, evaluation of the patient's stool for blood and purulent exudate can indicate the patient's response to therapy. Treatment results in improvement of one or more symptoms or signs of the intestinal inflammation.

## Claims

1. The use of IL-10 in the manufacture of a pharmaceutical composition for treating inflammatory bowel disease.

2. The use of claim 1 in which the inflammatory bowel disease is ulcerative colitis.

3. The use of Claim 1 in which the inflammatory bowel disease is Crohn's Disease.

4. The use of any preceding claim wherein the IL-10 is human IL-10.

## Patentansprüche

1. Verwendung von IL-10 bei der Herstellung einer pharmazeutischen Zusammensetzung zur Behandlung von entzündlicher Darmerkrankung.

2. Verwendung gemäß Anspruch 1, wobei es sich bei der entzündlichen Darmerkrankung um Colitis ulcerosa handelt.

3. Verwendung gemäß Anspruch 1, wobei es sich bei der entzündlichen Darmerkrankung um Morbus Crohn handelt.

4. Verwendung gemäß einem der vorstehenden Ansprüche, wobei es sich bei dem IL-10 um Human-IL-10 handelt.

## Revendications

1. Utilisation d'IL-10 dans la fabrication d'une composition pharmaceutique pour traiter une maladie inflammatoire de l'instestin.

2. Utilisation selon la revendication 1 dans laquelle la maladie inflammatoire de l'intestin est une colite ulcéreuse.

3. Utilisation selon la revendication 1 dans laquelle la maladie inflammatoire de l'intestin est la maladie de Crohn.

4. Utilisation selon l'une quelconque des revendications précédentes, dans laquelle l'IL-10 est de l'IL-10 humaine.
